# EUROPEAN PATENT SPECIFICATION

(11) **EP 2 416 815 B1**
(45) Date of publication and mention of the grant of the patent: **16.12.2015**
(21) Application number: 10762506.3
(22) Date of filing: 09.04.2010
(51) Int. Cl.: A61L 33/00, B05D 3/00, A61L 27/34, A61L 27/54, A61L 27/12, A61L 33/12

(54) **LIGAND-SPECIFIC INHIBITION OF ATTACHMENT OF IMMUNE CELLS TO IMPLANTABLE BIOMATERIALS**
LIGANDENSPEZIFISCHE HEMMUNG DER ANHAFTUNG VON IMMUNZELLEN AN IMPLANTIERBARE BIOMATERIALIEN
INHIBITION SPÉCIFIQUE DE LIGAND DE LA FIXATION DE CELLULES IMMUNITAIRES À DES BIOMATÉRIAUX IMPLANTABLES

(30) Priority: 05.10.2009 US 248618 P; 09.04.2009 US 167991 P
(43) Date of publication of application: 15.02.2012
(73) Proprietor: The Children's Hospital of Philadelphia, Philadelphia, PA 19104 (US); The Trustees Of The University Of Pennsylvania, Philadelphia, PA 19104 (US)
(72) Inventor: STACHELEK, Stanley, J., Philadelphia PA 19104 (US); DISCHER, Dennis, Philadelphia PA 19104 (US); LEVY, Robert, J., Merion Station PA 19066 (US); TSAI, Richard, Philadelphia PA 19146 (US)
(74) Representative: Schwabe - Sandmair - Marx
(86) International application number: PCT/US2010/030558
(87) International publication number: WO 2010/118335

(56) References cited:
- US-A- 5 840 083
- US-A- 6 033 719
- J. A. Jones ET AL: "Macrophage behavior on surface-modified polyurethanes.", , 21 January 2004 (2004-01-21), XP55098931, Retrieved from the Internet: URL:http://www.dsm.com/content/dam/dsm/med ical/en_US/documents/ta-02-06-macrophage-b ehavior-on-surface-modified-polyurethanes. pdf [retrieved on 2014-01-28]
- ANDERSON ET AL.: 'Foreign Body Reaction To Biomaterials' SEMIN IMMUNOL. vol. 20, no. 2, 01 April 2008, pages 86 - 100, XP022520625
- TAKIZAWA ET AL.: 'Macrophage Tolerance: CD47-SIRP-Mediated Signals Matter.' NATURE IMMUNOLOGY. vol. 8, no. 12, 01 December 2007, pages 1287 - 1289, XP055094296
- HAN ET AL.: 'CD47, A Ligand for the Macrophage Fusion Receptor, Participates in Macrophage Multinucleation.' JOURNAL OF BIOLOGICAL CHEMISTRY vol. 275, no. 48, 01 December 2000, pages 37984 - 37992, XP000941936
- IDE ET AL.: 'Role For CD47-SIRP-a Signaling In Xenograft Rejection By Macrophages.' PROCEEDINGS OF THE NATIONAL ACADEMY OF SCIENCES vol. 104, no. 12, 20 March 2007, pages 5062 - 5066, XP055094299

## Description

### RELATED APPLICATIONS

This application claims priority under 35 U.S.C. § 119 to U.S. Provisional Application No. 61/248,618, filed on October 5, 2009, and U.S. Provisional Application No. 61/167,991, filed on April 9, 2009, the contents of both of which are incorporated by reference herein, in their entirety and for all purposes.

### FIELD OF THE INVENTION

The invention relates generally to the field of immunology. More particularly, the invention relates to protecting biomaterials from immune cell-mediated damage, especially when the biomaterials are implanted into the body of an animal.

### BACKGROUND OF THE INVENTION

Various publications, including patents, published applications, technical articles and scholarly articles are cited throughout the specification. Each of these cited publications is incorporated by reference herein, in its entirety and for all purposes.

Material degradation of implantable polyurethane (PE) containing devices, as a result of chronic inflammation, remains an important problem in biomaterials research. Reactive oxygen species (ROS) released from adherent monocyte-derived macrophages (MDMs) play a role in the structural damage observed in PE-containing vascular implants such as pacemaker lead insulation (Schubert, MA et al. (1997) J. Biomed. Mater. Res. 34:519-30; Labow, RS et al. (2002) Biomaterials 23:3969-75; and, Santerre, JP et al. (2005) Biomaterials 26:7457-70). Modifying bulk preparations of PE with phenol-based anti-oxidants effectively reduces the ROS mediated damage to PE films, but is limited by the fact that the anti-oxidant capacity is expended after reacting with the oxygen radical (Stachelek, SJ et al. (2006) J. Biomed. Mater. Res. A. 78:653-61; Stachelek, SJ et al. (2007) J. Biomed. Mater. Res. A. 82:1004-11; Schubert, MA et al. (1996) J. Biomed. Mater. Res. 32: 493-504; and, Schubert, MA et al. (1997) J. Biomed. Mater. Res. 34:493-505).

Previous attempts to inhibit immune cell activation have largely used a co-blending strategy to modify the PE surface by adding to it molecules such as polyethylene oxide (PEO), polypropylene oxide (PPO) or tri-block fluorinated macromolecules with the idea that the surface-oriented molecules will reduce protein adsorption (Massa, TM et al. (2007) J. Biomed. Mater. Res. A. 81:178-85; Ward, R et al. (2007) J. Biomed. Mater. Res. A. 80:34-44; and, Ebert, M et al. (2005) J. Biomed. Mater. Res. A. 75:175-84). Although these co-blending strategies did indeed reduce the levels of MDM activation, co-blending as a delivery strategy is limited, in part, because co-blending is inefficient, as not all the therapeutic molecules will be oriented on the PE film surface where it is needed; and, co-blending alters the physical properties of the PE film.

Jones at al. (J. Biomater. Sci. Polymer, vol 15, p. 567-584, 2004) studies the possibility to modifiy polyurethanes with fluorocarbon, polyethylene oxide and poly(dimethylsiloxane).

There exists a need in the art for more efficient protection of biomaterials which does not alter the physical properties of the biomaterials.

### SUMMARY OF THE INVENTION

The invention provides a method for inhibiting or reducing immune cell attachment to a biomaterial, comprising attaching CD47 or a subdomain thereof that is capable of binding to SIRP-alpha to the surface of the biomaterial, wherein the CD47 or the subdomain thereof that is capable of binding to SIRP-alpha inhibits or reduces immune cell-mediated damage to the biomaterial. The methods can be used to inhibit or reduce immune cell attachment or immune cell-mediated damage to biomaterial *in vitro* and *in vivo*.

The invention also provides biomaterials protected from immune cell attachment and/or immune cell-mediated damage to the biomaterials. A protected biomaterial comprising CD47 or the subdomain thereof that is capable of binding to SIRP-alpha attached to the surface of the biomaterial. The biomaterial further comprises at least one linking molecule on the surface of the biomaterial. The linking molecule mediates the attachment of the CD47 or the subdomain thereof that is capable of binding to SIRP-alpha to the surface.

Preferred biomaterials comprise a polymer such as a polypropylene, polyethylene, polystyrene, polymethylmethacrylate, polyurethane, polyfluorotetraethylene, or polyvinyl, or mixtures thereof. The methods can be used to protect biomaterials in an implant, catheter, medical device, tube, or therapeutic agent delivery vehicle, among other things. Thus, implants, catheters, medical devices, tubes, or therapeutic agent delivery vehicles can comprise one or more biomaterials, and/or can be coated with one or more biomaterials.

The surface of the biomaterial can be modified to comprise at least one linking molecule to mediate interaction of the biomaterial with CD47 or the subdomain thereof that is capable of binding to SIRP-alpha. In addition, the CD47 or the subdomain thereof that is capable of binding to SIRP-alpha can be complexed with at least one

The described methods can be used to protect biomaterials from any immune cell attachment or damage induced by any immune cell. Preferably, the immune cell expresses SIRP-α. Exemplary immune cells include monocytes and macrophages, as well as polymorphonuclear cells, for example, neutrophils. The damage inhibited or reduced by the inventive methods is directly related to the inflammatory response, such as, but not limited to cytokines, reactive oxygen species, or hydrolytic enzymes released by immune cells that can have a deleterious effect upon the host and/or the implanted biomaterial.

The invention also provides kits for protecting biomaterials. Generally, the kits comprise CD47 or the subdomain thereof that is capable of binding to SIRP-alpha and instructions for using the kit in a method to protect biomaterials and/or a method to inhibit or reduce immune cell attachment to biomaterials. The kits can comprise one or more linking molecules capable of being attached to the surface of the biomaterial or to CD47 or or the subdomain thereof that is capable of binding to SIRP-alpha. Examples of linking molecules include avidin, biotin, thiol, folate, the folate receptor, SPDP, and SMCC. The CD47 or the subdomain thereof that is capable of binding to SIRP-alpha can be pre-complexed with a linking molecule.

### BRIEF DESCRIPTION OF THE DRAWINGS

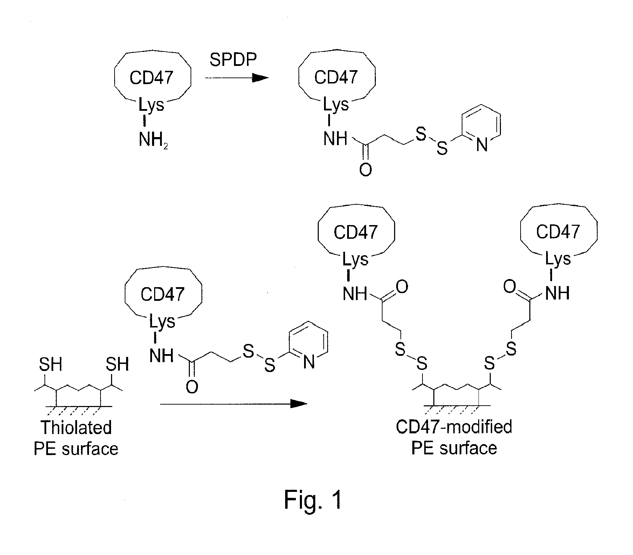

Figure 1 shows a chemical scheme for appending CD47 to a photoactivated polyurethane surface using the bifunctional-crosslinker SPDP.

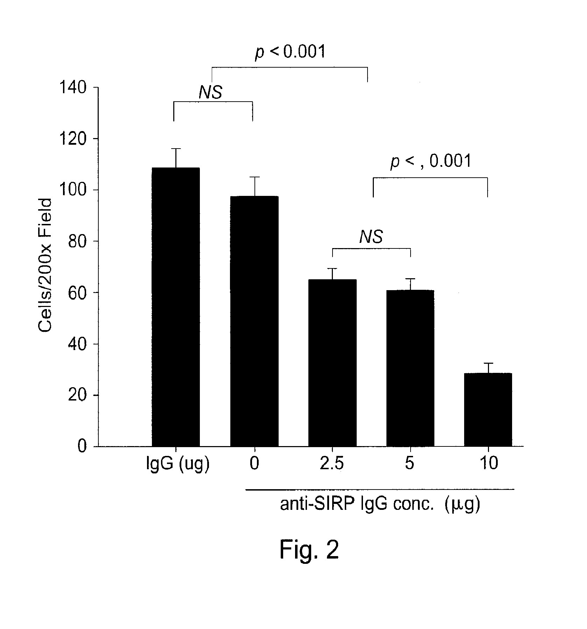

Figure 2 shows SIRP-α is involved in MDM attachment mechanisms to PE. Human MDM cells from the THP-1 line were activated with phorbol esters and cultured on PE films in the presence of increasing concentrations of anti-SIRP-α antibody. After 48 hours, films were washed and assessed for adherence to the PE film. These data show that anti-SIRP blocks attachment of THP-1 cells to PE films.

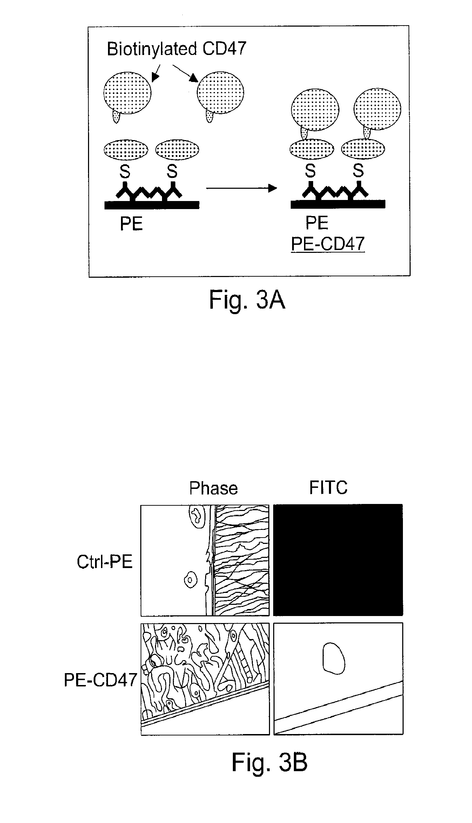

Figure 3A and 3B show immobilization of CD47 on PE surfaces. (A) shows a schematic diagram illustrating the immobilization of biotinylated CD47 on PE films by reaction with surface immobilized avidin. (B) shows an anti-CD47 antibody and a FITC-conjugated species-specific antibody used for immunofluorescence detection of immobilized CD47 showing robust signal in CD47 immobilized PE surfaces.

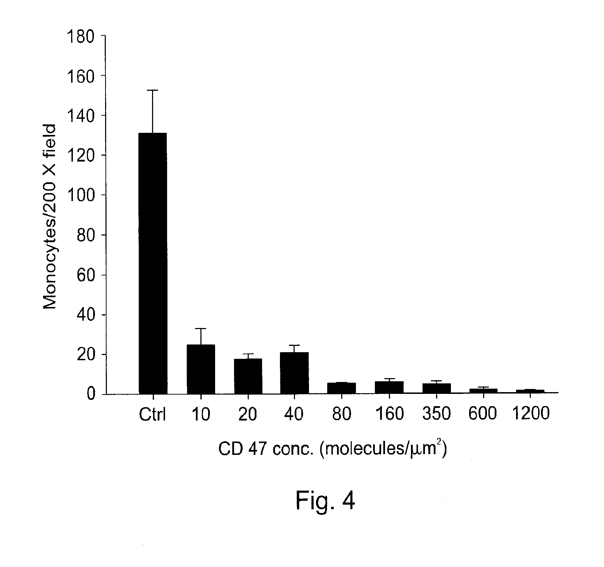

Figure 4 shows surface-immobilized CD47 inhibits MDM binding to PE. PE surfaces were modified by immobilizing increasing concentrations of biotinylated CD47 onto the surface via photo-reactive chemistry. Cells of the human MDM cell line, THP-1, were activated with phorbol esters and cultured on the CD47-modified PE films. After 48 hours, films were washed and assessed for adherence to the PE film THP-1 cells were cultured on the modified films for 48 hours. Adherence of THP-1 cells to PE surfaces was significantly inhibited by the presence of immobilized CD47.

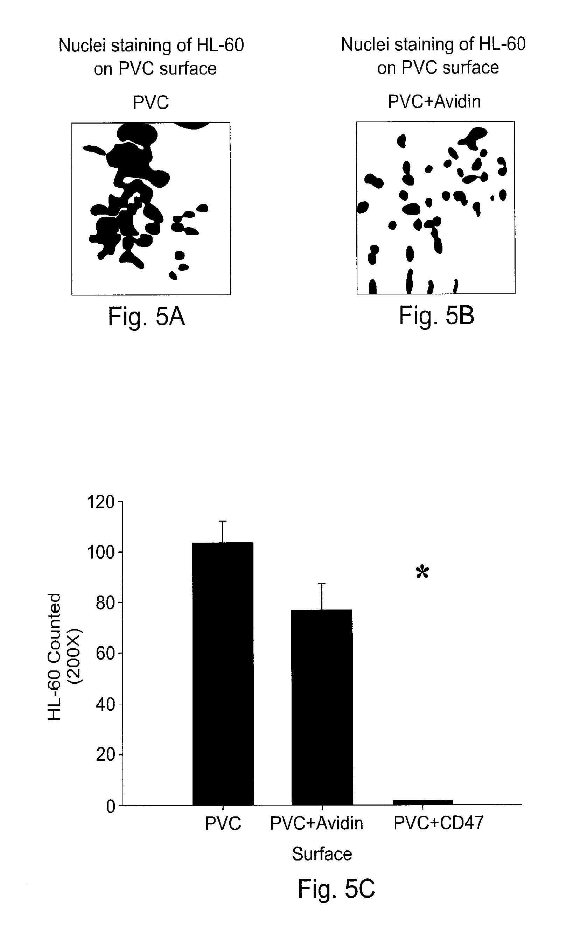

Figures 5A-5C show nuclear staining of HL-60 neutrophils on the surface of PVC tubes. PVC tubes were modified by immobilizing CD47 on the surface. Cells of the neutrophil line, HL-60, were placed in surface modified and control PVC tubes and incubated for several hours. (A) shows DAPI-stained neutrophils adhered to unmodified PVC tubing. (B) shows DAPI-stained neutrophils adhered to PVC tubing with avidin immobilized on its surface. (C) shows the quantification of the number of DAPI-stained neutrophils that adhered to the surface of unmodified PVC, PVC modified with avidin alone, and PVC modified with avidin and biotinylated CD47.

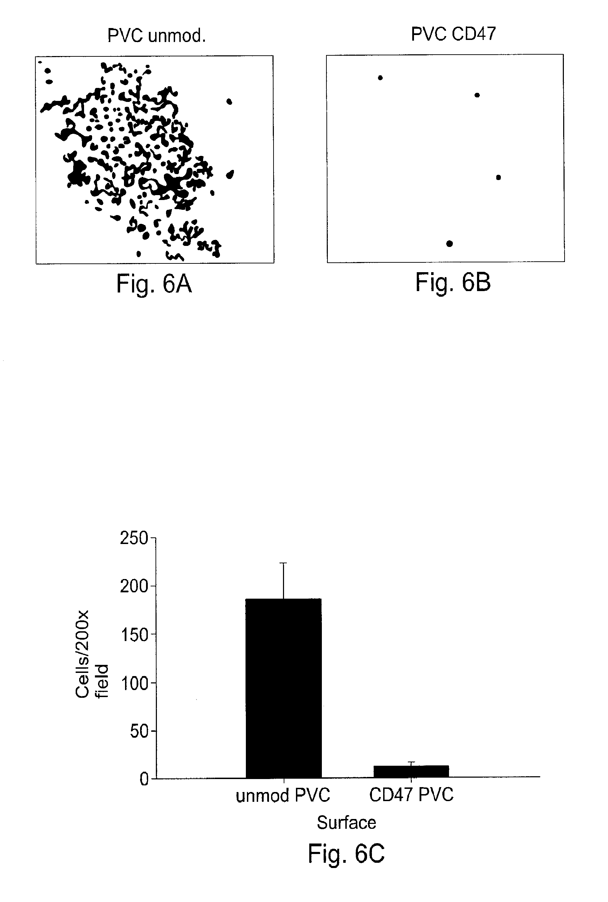

Figures 6A-6C show human white blood cells on the surface of PVC tubes after simulated blood flow. PVC tubes were modified by immobilizing CD47 on the surface. Freshly isolated human whole blood was placed in surface modified and control PVC tubes, and blood flow was simulated using a Chandler loop system for several hours. (A) shows DAPI-stained leukocytes adhered to unmodified PVC tubing. (B) shows minimal DAPI-stained leukocytes on the surface of PVC tubing with CD47 immobilized on its surface. (C) shows the quantification of the number of DAPI-stained leukocytes that adhered to the surfaces of unmodified PVC and PVC modified with avidin and biotinylated CD47.

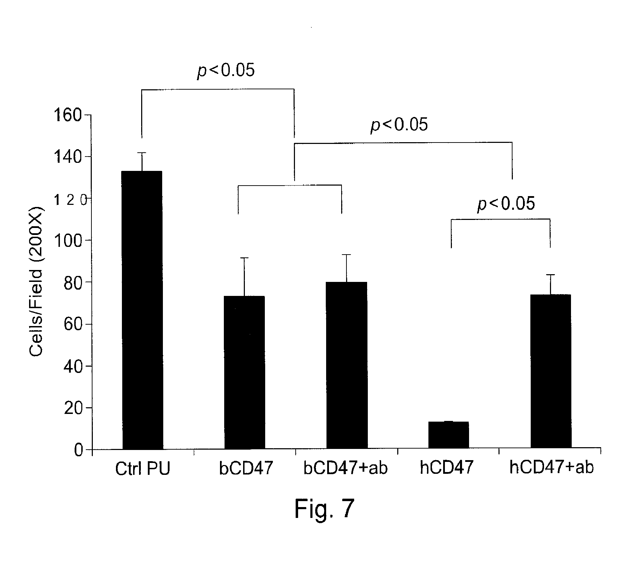

Figure 7 shows the quantification of the number of MDM cells that adhered to the surfaces of unmodified polyurethane (PU), PU modified with bovine CD47 (bCD47), PU pre-incubated with anti-hCD47 antibody B6H12 and modified with bCD47, PU modified with human CD47 (hCD47), and PU pre-incubated with B6H12 and modified with hCD47.

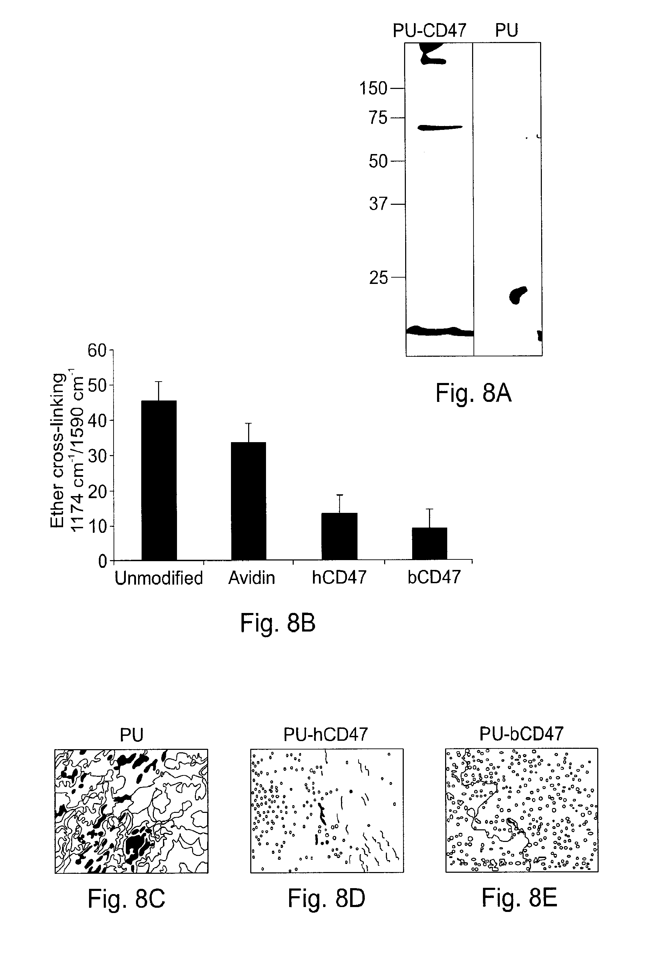

Figures 8A-8E show assessment of oxidative related degradation of polyurethane (PU) subdermal implants in rats. (A) shows the presence of human CD47 (hCD47) on the explanted films. (B) shows ether cross-linking on the explanted films. (C), (D) and (E) show scanning electron micrographs of the explanted films

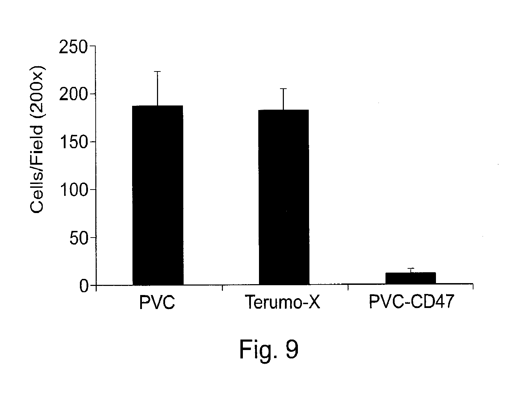

Figure 9 shows human white blood cells on CD47 modified surfaces of (A) polyvinyl chloride (PVC) tubing and (B) Terumo-X tubing after simulated blood flow.

### DETAILED DESCRIPTION OF THE INVENTION

It has been observed in accordance with the present invention that CD47 is capable of conferring protection to biomaterials against damage caused by immune cells by means of utilizing the CD47-SIRPα pathway. The feasibility of passivating polyurethane and polyvinyl chloride surfaces with the Ig domain of CD47 was investigated as a means of reducing the inflammatory response initiated against such biomaterial polymers. Accordingly, the embodiments of the invention provide methods for protecting a biomaterial. In general, the methods comprise attaching CD47, the subdomain thereof that is capable of binding to SIRP-alpha or other suitable domain or subdomain thereof, to the surface of a biomaterial, wherein the CD47, or the subdomain thereof that is capable of binding to SIRP-alpha inhibits or reduces immune cell-mediated damage to the biomaterial.

Signal regulatory protein alpha (SIRP-α) is a potential signaling protein found in MDMs that may be targeted to confer immunoresistance to implantable biomaterials. SIRP-α is a transmembrane protein expressed by MDMs and other cells of myeloid origin. SIRP-α signaling is mediated by tyrosine inhibitory motifs (ITIMs) located in the cytoplasmic tail of SIRP-α. The SIRP ITIMs activate Src homology domain 2-containing phosphatases -1 (SHP-1) and (SHP-2). Recruitment and activation of SHP-1 and SHP-2 by SIRP-α negatively regulates phagocytosis by macrophages (van Beek, EM et al. (2005) J. Immunol. 175:7781-7). SIRP-α may also be identified as CD172A, SHPS1, P84, MYD-1, BIT, PTPNS1 or SIRP-1α.

CD47, also known as integrin associated protein, is a ubiquitously expressed transmembrane protein. It is a member of the immunoglobulin (Ig) superfamily of membrane proteins with a single, variable Ig domain at its N terminus. The Ig domain of CD47 has been recently identified as a ligand of SIRP-α (Brown, EJ et al. (2001) Trends Cell Biol 11:130-5; Vernon-Wilson, EF et al. (2000) Eur. J. Immunol. 30:2130-7; Takizawa, H et al. (2007) Nat. Immunol. 8:1287-9; and, Subramanian, S et al. (2006) Blood 107:2548-56). Additional studies have shown that SIRP-α binding to CD47 prevents phagocytosis of CD47 immobilized microbeads and red blood cells, in a species specific manner, by MDMs (Subramanian, S et al. (2006) Blood 107:2548-56; Oldenborg, PA (2004) Leuk, Lymphoma 45:1319-27; Tsai, RK et al. (2008) J. Cell Biol. 180:989-1003; Oldenborg, PA et al. (2000) Science 288:2051-4; and, Okazawa, H et al. (2005) J. Immunol. 174:2004-11). Furthermore, it has been shown that a CD47 homologue expressed in Myxoma virus contributes to the downregulation of MDM activation (Cameron, CM et al. (2005) Virology, 337:55-67). The Ig domain of the CD47 protein is highly variable between species. It has been reported that this variability affects the binding affinity between SIRP-α and CD 47 (Subramanian, S. et al. (2007) J. Biol. Chem. 282(3):1805-18).

The methods described and exemplified herein are suitable for protecting any biomaterial. Biomaterials include any materials suitable for biological, biomedical, or medical applications. Non-limiting examples of biomaterials include fabrics, ceramics, polymers, thermoplastics such as polyaryletherketone and polyetherketoneketone, adhesives, bone cement, metals, and the like. Polymers are most preferred. Biomaterial polymers include, without limitation, polypropylene, polyethylene, polyester, polystyrene, polymethylmethacrylate, polyurethane, polyfluorotetraethylene, or polyvinyl, polyethyleneimine, polyamide, polyacrylonitrile, polyacrylate, polymetacrylate, polyorthoester, polyether-ester, polylactone, polyalkylcyanoacrylate, polyethylenvinyl acetate, polyhydroxybutyrate, polytetrafluoroethylene, polyethylene terephthalate, polyoxyethylene, and the like, or mixtures thereof. Highly preferred polymers include polyurethane and polyvinyl chloride.

Biomaterials are used in various biological, biomedical, or medical applications. Such applications include the make-up of compositions, products, and devices such as artificial joints, implants, stents, dental implants, bone cement, catheters, tubes, artificial tendons and ligaments, artificial skin, artificial heart valves, delivery vehicles for therapeutic agents, particles, and the like. In preferred aspects, the methods are applicable to protect the biomaterials used to fabricate these compositions, products, and devices and/or biomaterials coated onto the surface of these compositions, products, and devices. Thus, in preferred aspects, the methods are applicable to protect compositions, products, and devices comprising biomaterials. Preferably, the methods are used to protect implants, tubes, catheters, and therapeutic agent delivery vehicles comprising biomaterials. More preferably, the methods are used to protect leads of a cardiac pacemaker comprising biomaterials and tubes comprising biomaterials that carry blood between a patient and a heart-lung bypass machine.

CD47 plays a role, among other things, in cell adhesion to the extracellular matrix *in vivo,* and also serves as a receptor for the C-terminal domain of thrombospondin. CD47 is also believed to play a role in cellular signal transduction. The described methods can utilize CD47, or any isoform thereof. The methods of the invention can also utilize the any suitable subdomain of CD47, including the extracellular Ig domain or subdomain thereof. Suitable subdomains of CD47 or its Ig domain preferably will be those that are capable of binding to or otherwise interacting with SIRP-α or any other ligand on an immune cell that signals the immune cell not to activate or produce biomaterial-damaging agents such as reactive oxygen species.

The CD47 or the subdomain thereof that is capable of binding to SIRP-alpha can be from any species, including mouse, rat, rabbit, horse, pig, sheep, cow, cat, dog, human and the like. Porcine, bovine and human CD47 are particularly preferred.

Biomaterials can be modified to reduce or inhibit immune cell attachment and/or immune cell-mediated damage to the biomaterials. For example, a modified biomaterial comprises CD47 or the subdomain thereof that is capable of binding to SIRP-alpha attached to the surface of the biomaterial.

The methods are suitable to inhibit or reduce immune cell-mediated damage to biomaterial *in vitro,* for example, biomaterials used in cell culture or in experiments generally. The methods are also suitable to inhibit or reduce immune cell-mediated damage to the biomaterial *in vivo,* for example, biomaterials permanently or temporarily implanted, administered to, inserted, or otherwise inside of an animal.

The CD47 or the subdomain thereof that is capable of binding to SIRP-alpha can be attached to the biomaterial according to any means suitable in the art. For example, the CD47 or the subdomain thereof that is capable of binding to SIRP-alpha can be mixed with the biomaterial during manufacture of a composition, product, or device comprising the biomaterial such that the CD47, Ig domain, or suitable subdomain thereof is interspersed throughout the biomaterial, including on the surface of the particular composition, product, or device produced from the biomaterial. In some aspects, the CD47 or the subdomain thereof that is capable of binding to SIRP-alpha can be attached to the biomaterial, for example, by non-covalent intermolecular attractions, or by ionic or covalent bonds between the biomaterial and the CD47 or the subdomain thereof that is capable of binding to SIRP-alpha

In some preferred aspects, the CD47 or the subdomain thereof that is capable of binding to SIRP-alpha can be attached to the biomaterial by way of linking molecules. The linking molecules can be complexed or conjugated to the biomaterial and/or the CD47 or the subdomain thereof that is capable of binding to SIRP-alpha. Linking molecules are any molecules capable of mediating or facilitating the attachment of the CD47 or the subdomain thereof that is capable of binding to SIRP-alpha to the biomaterial. Linking molecules can be any organic or inorganic chemical, proteins, polypeptides, polynucleotides, polysaccharides, lipids, thiols, and the like. Linking molecules are known in the art, and can be selected according to the needs of the practitioner. Some non-limiting examples of linking molecule pairs include avidin or streptavidin and biotin, thiol and Succinimidyl 3-(2-pyridyldithio)-propionate (SPDP) or Succinimidyl 4-[N-maleimidomethyl]cyclohexane-1-carboxylate (SMCC), or suitable variants or isoforms thereof, and folate and the folate receptor.

Thus, in some preferred aspects, CD47 can be prepared comprising thiol-reactive groups via reacting NH₂ of its lysine residues with bifunctional amino- and thiol-reactive cross-linkers such as SPDP (as shown in Figure 1, inserting 2-pyridyldithio groups) or N-succinimidyl trans-4-maleimidomethylcyclohexanecarboxylate (SMCC, resulting in the thiol-reactive maleimide residues attached to the protein). The thiol-reactive CD47 can then react with a thiol group on the biomaterial surface. As a result of this interaction, the surface of biomaterial will be coated with a layer of CD47, bound to the biomaterial macromolecules via covalent bonds (Figure 1). In some preferred aspects, biotinylated CD47 can be attached to the biomaterial surface via avidin as described and exemplified herein, for example, as shown in Figure 3A. In some preferred aspects, methodologies which include, but are not limited to plasma glow discharge or nucleophilic substitution can be used to attach CD47 to the biomaterial surface.

The inventive methods protect biomaterials from damage induced or exacerbated by immune cells. Preferably, the methods protect biomaterials from damage induced or exacerbated by polymorphonuclear cells, including neutrophils, basophils, and eosinophils, peripheral blood mononuclear cells, including monocytes, and/or macrophages. In some aspects, the methods protect biomaterials from damage induced or exacerbated by immune cells that express a ligand or receptor for CD47, such as SIRP-α. In some aspects, the methods can protect against an immune cell response caused by cis-acting CD47-SIRP-α mechanisms, elicited by the CD47 molecular interactions with SIRP-α molecules that are both expressed in the same cell. The methods can protect against any damage induced or exacerbated by immune cells, and preferably protect against oxidative damage, such as the damage to the biomaterial caused by exposure to reactive oxygen species and radicals produced by immune cells. The methods can also protect against inflammatory molecules, cytokines and/or hydrolytic enzymes produced by immune cells.

Embodiments of the invention include kits for protecting biomaterials utilizing the methods described and exemplified herein. In some aspects, the kits comprise CD47 or the subdomain thereof that is capable of binding to SIRP-alpha and instructions for using the kit in a method for protecting a biomaterial. In some aspects, the kits further comprise one or more linking molecules capable of being attached to the surface of a biomaterial, and/or capable of being attached to the CD47 or the subdomain thereof that is capable of binding to SIRP-alpha and may further comprise reagents suitable for attaching the linking molecule to the CD47 or the subdomain thereof that is capable of binding to SIRP-alpha. Some aspects, the CD47, Ig domain, or subdomain thereof is complexed with a linking molecule. In some detailed aspects, the CD47 or the subdomain thereof that is capable of binding to SIRP-alpha is complexed with SPDP or SMCC. In some detailed aspects, the CD47 or the subdomain thereof that is capable of binding to SIRP-alpha is complexed with biotin. In some detailed aspects, the CD47 or the subdomain thereof that is capable of binding to SIRP-alpha is complexed with folate. In some detailed aspects, the CD47 or the subdomain thereof that is capable of binding to SIRP-alpha is complexed with the folate receptor.

The following examples are provided to describe exemplary aspects of the invention in greater detail. They are intended to illustrate, not to limit, the invention.

### EXAMPLE 1

### The SIRP-α Pathway is Involved in MDM Binding to PE

To begin to assess the contribution of the SIRPα/CD47 pathway in MDM attachment to PE surfaces, cells of the MDM cell line (THP-1) were transduced with the addition of 1.6 x 10⁻⁷ M of phorbol 12 myristate 13-acetate (PMA) to the media and were cultured on PE films in the presence of increasing concentrations (0, 2.5, 5, 10 µg) of anti-SIRP-α antibody directed against the protein's extracellular domain. Cells were allowed to attach to the PE films for 48 hours, at which time the films were washed with PBS and the remaining cells were fixed with 4% paraformaldehyde and stained with the nuclei specific stain DAPI.

Cell retention was determined by DAPI staining and counting the 9 separate 200X fields. As shown in Figure 2, transformed THP-1 attachment to PE surfaces was not affected by the presence of 10 µg of non-specific IgG antibody (96±7.9 cells/200 x field vs. 108±7.7 cells/200X field). However, binding was significantly (p < 0.001) inhibited by the presence of 2.5 µg (64.4 ± 4.7 cells/200 x field) or 5 µg (60.5 ± 4.7 cells/200 x field) of SIRP-α blocking antibody. There was a 2-fold further reduction in THP-1 binding to PE in the presence of 10 µg of anti-SIRP-α blocking antibody. These results indicate a SIRPα dependent mechanism in THP-1 binding to PE surfaces.

### Example 2

### Characterization of CD47 Immobilization on PE Surfaces

In the initial studies, biotinylated CD47 of human origin (hCD47) was appended to avidin-immobilized films, as shown in Figure 3. All studies were done using the photoactivation chemistry. This chemistry is specific for linking the avidin to the polymer surface (*e.g.*, PVC or PE). Once the avidin is linked the biotinylated CD47 can then be reacted with the avidin. Later studies used a novel surface modification involving a polymeric photo cross-linker (PDT-BzPh) composed of 2-pyridyldithio groups (PDT) linked to the benzophenone (BzPH) photoreactive groups (Chorny, M et al. (2006) Mol. Ther. 14:382-91).

Biotinylated CD47 was immobilized to the PE surface by applying PDT-BzPh as a micelle suspension. Under UV-irradiation, BzPh groups form covalent bonds with PE macromolecules. PDT groups on the PE films are then reduced to thiol-groups by reacting with a solution of TCEP. Avidin (10mg/ml) was reacted with SPDP and purified by passing through a Sephadex^{®} (GE Healthcare Bio-Sciences AB LLC, Uppsala Sweden) column. PE films were reacted with the thiol reactive avidin and incubated overnight at room temperature. The avidin-immobilized films were then washed 5 times in dH₂O and increasing concentrations (3.9 ng, 7.8 ng, 15.62 ng, 31.25 ng, 62.5 ng, 125 ng, 250 ng, and 500 ng) of biotinylated CD47 (see below for details) were added to each film.

To assess the immobilization of CD47 on the PE surface, a control film and avidin-crosslinked films were incubated with mouse anti-CD47 antibody (B6H12 directed against the extracellular domain of human CD47). The antigen antibody complex was detected by incubation with a FITC-conjugated mouse secondary antibody. The immune complex was visualized using fluorescent microscopy and a FITC filter set.

A representative fluorescent photomicrograph shows robust fluorescence (Figure 3B) in CD47 immobilized surfaces that is absent from the control PE films indicating that the CD47 was indeed immobilized to the PE surface. Fluorescent labeling of CD47 immobilized surfaces with FITC-conjugated anti-CD47 antibody showed that a range (0-500 ng) of biotinylated CD47 loaded onto the photoactivated PE surfaces corresponded to a CD47 surface concentration range of 0-1200 molecules CD47/µm².

Source of biotinylated CD47. Plasmids encoding the extracellular domain of hCD47 were PCR amplified and ligated in frame with the vector pEF-BOS-XB which formed the in frame fusion of CD4d3+4 biotin at the C-terminus of the extracellular domain of CD47. This construct was transfected into CHO (-K1) cells and the secreted CD47-CD4d3 + 4 was concentrated, biotinylated at the C-terminus, and dialyzed. The protein was affinity purified using a monomeric avidin and dialyzed against PBS.

### Example 3

### Immobilized CD47 Inhibits MDM Attachment to Polyurethane Elastomers

PMA-activated THP-1 cells (100,000 cells) were seeded on 1 cm² PE-CD47 (over a range of CD47 concentrations) and control films. PE-CD47 films were prepared with biotinylated hCD47 as described in Example 2. Cells were allowed to attach to the PE films for 48 hours, at which time the films were washed with PBS and remaining cells were fixed with 4% paraformaldehyde and stained with the nuclei-specific stain DAPI. Cell retention was determined by DAPI staining and counting 9 separate 200X fields. Figure 4 shows profound inhibition of THP-1 cell attachment to CD47 immobilized surfaces.

At the lowest CD47 surface concentration (∼ 10 molecules/µm²) there was significant (p < 0.001) reduction of THP-1 cell attachment (24.6 ± 8.2 cells/200 X field) compared to unmodified control surfaces (131.23 ± 21.6 cells/200 X field). There was a further four-fold reduction in THP-1 attachment to PE-CD47 surfaces at CD47 concentrations between 80-301 molecules/µm². Cell attachment was virtually abolished at CD47 concentrations greater than 600 molecules/µm². These data show that immobilized CD-47 on PE reduced monocyte attachment and provide proof of principal for the feasibility of establishing a biomimetic PE surface with immobilized CD 47 molecules.

### Example 4

### Inhibition of neutrophil attachment to CD47 coated polyvinylchloride (PVC) cardiopulmonary bypass tubing

Studies were conducted to determine if surface immobilized, recombinant human CD47 (biotinylated) can inhibit neutrophil adhesion to polyvinyl chloride (PVC) surfaces.

Briefly, cells cultivated from the human neutrophil cell line, HL-60, were suspended at a concentration of approximately 1.5 x 10⁶ cells/ml in growth media supplemented with 5 µg/ml of IgG and the phorbol ester, phorbol 12-myristate 13-acetate (PMA). The resuspended cells were added to uncoated (control) PVC tubing or PVC tubing that was modified by immobilizing CD47 (via avidin-biotin affinity) on the surface of the tubing, or with a coating of avidin alone (avidin control). The tubes containing the cells were capped at both ends and shaken for 3 hours at 37°C. After this incubation period, the cell media containing unbound cells was removed, the tubing was washed 4x with PBS, and attached cells were fixed by the addition of 4% paraformaldehyde. Cells attached to the PVC tubing were quantified by staining with the fluorescent dye DAPI (blue color on fluorescent micrographs) and visualized using a fluorescent microscope with the appropriate filter set.

Fluorescent micrographs of the cells attached to PVC tubing are shown in Figure 5. Figure 5 demonstrates robust cell retention, as evidenced by ample DAPI (blue) staining, on unmodified PVC surfaces (Figure 5A) or PVC surfaces modified with avidin alone (Figure 5B). In contrast, HL-60 attachment to surfaces coated with immobilized CD47 demonstrated little to no cell attachment. These images, which have no DAPI staining are not shown. Quantitative analyses of these data showed that HL-60 attachment to PVC was somewhat inhibited by the presence of surface immobilized avidin (Figure 5C). However, these results were not significantly different from the PVC control data (Figure 5C). The surface attachment of CD47 to the PVC bypass tubing substantially blocked HL-60 attachment to the PVC tubing, as shown in Figure 5C.

These results strongly suggest that CD47 immobilized on the surface of a biomaterial polymer can reduce or prevent the acute inflammatory response caused by polymorpholeukocyte attachment to PVC tubing, a typical tubing used in cardiopulmonary bypass systems.

### Example 5

### Inhibition of white blood cell attachment to CD47 modified polyvinyl chloride (PVC) tubing in a cardiopulmonary bypass simulation model using fresh human whole blood

The Chandler loop is an apparatus in which a moving column of blood (or suspended cells in culture) flows in a circular closed tubing loop that is rotated on a slanted, electrically driven turntable. Investigations involving the simulation of blood flow within a cardiopulmonary bypass (CPB) circuit often use the Chandler loop. In this Example, the Chandler loop was used to investigate the effects of CD47 toward protecting PVC surfaces from whole blood under simulated blood flow conditions.

Briefly, 10 ml of whole human blood were freshly drawn (IRB-approved protocol), supplemented with sodium citrate to inhibit clotting, and introduced to a Chandler loop prepared from PVC tubing (approximately 33 cm in length) which was either surface modified with immobilized CD47 (biotinylated) or unmodified (no CD47, and no avidin (control)). The tubing was rotated for three hours at 37°C, with resulting circulation of the blood within the loop. At the end of the protocol, the blood was removed, and the tubing was washed with phosphate buffered saline. Attached cells were fixed with 4% paraformaldehyde.

Five segments of tubing (approximately 3 cm long), located at regular intervals along the length of the tubing, were excised, and any attached cells were quantified by staining with the fluorescent dye DAPI (blue color on fluorescent micrographs) and visualized using a fluorescent microscope with the appropriate filter set. The results are shown in Figure 6.

Figure 6A shows robust cell attachment, reflecting white blood cell adhesion on the surface of the unmodified, control PVC tubing. In contrast, there was little cellular attachment to the PVC tubing having CD47 immobilized on its surface (Figure 6B). Quantitative analysis showed a 20-fold greater cellular attachment in unmodified PVC tubing (control) relative to the CD-47 surface-modified PVC tubing (Figure 6C).

These data obtained with fresh whole human blood support the data shown in Example 4 obtained from using the neutrophil cell line HL-60 cells. In addition, these results show that the anti-inflammatory properties of surface bound CD47 on PVC tubing are fully functional in a model that approximates the cardiopulmonary bypass circuit, which is one example of a clinical application for this technology.

### Example 6

### Porcine Implant Model for the Biological Response to Right-Sided Transvenous Pacer Lead Insulation Fabricated from Polyurethane ± CD47

This is a prophetic example.

Cracking of explanted pacemaker lead insulation has been widely noted (Wiggins, MJ et al. (2001) J. Biomed. Mater. Res. 58:302-7; and, Sutherland, K et al. (1993) J. Clin. Invest. 92:2360-7). Efforts to model this *in vivo* have largely relied upon the rat subdermal implant model, which is a useful and cost effective *in vivo* model to screen CD47-containing PE variants for resistance to oxidative degradation (Schubert, MA (1996) J. Biomed. Mater. Res. 32:493-504; Schubert, MA et al. (1997) J. Biomed. Mater. Res. 34:493-505; and, Stachelek, SJ et al. (2007) J. Biomed. Mater. Res. A 82:1004-11. However, PE degradation has never been studied in an *in vivo* model of transvenous pacer lead implantation.

Lead formulations of CD47-modified PE will be examined in a right sided transvenous implant model. The rationale for this model is as follows: 1. The transvenous implant is a clinically relevant model approximating the use of PE as a cardiac pace maker lead insulation used clinically. 2. The transvenous implant allows *in vivo* examination of the effects of a blood-contacting environment upon host inflammatory response to implanted PE.

A clinical application for antioxidant modified PE will be simulated by implanting PE tubing ± a candidate CD47 modification into porcine jugular veins for durations up to 10 weeks. With regard to oxidative degradation, it has been reported that 10 weeks *in vivo* corresponds to 2 week exposure in the solution of H₂O₂ CoCl₂ (Christenson, EM et al. (2004) J. Biomed. Mater. Res. A. 70:245-55; and, Christenson, EM et al. (2004) J. Biomed. Mater. Res. A. 269:407-16).

In brief, castrated male or non-pregnant female swine (Yorkshire Cross) will be anesthetized and intubated. A cut down to the jugular vein will allow the placement of PE tubing, whereupon it will be secured in the vein and the remaining portion will be secured in a loop and placed in a subcutaneous pocket.

Explant analysis. At the termination of the study, the animal will be euthanized following standard operating procedures. Leads will be explanted and shipped overnight for further analysis at the Children's Hospital of Philadelphia. Half of the length of the tubing will be processed for changes in the mechanical-physical properties as a result of implantation. The remaining half will be processed for biologically relevant endpoints.

Mechanical-Physical Properties. Explanted tubing will be washed in a detergent solution to remove biological debris. Soft segment chain scission and loss will be assessed via FTIR analysis. SEM will provide qualitative assessment of damage to the implanted PE surface. Uniaxial stress-strain tests will also be performed to determine any loss of elasticity as result of implantation.

Biological Endpoints. Half of the explanted tubing will be placed in formalin and assessed for relevant biological endpoints. Giemsa staining will be used to identify cell types that are present on the explanted PE tubing. DAPI staining will be used to quantify cell numbers on the explanted PE surfaces. Proteins of interest will be identified via immunofluorescence microscopy. These biological endpoints will correlate with the mechanistic endpoints as well as with the results obtained from initial *in vivo* studies of subdermal implants in rats.

### Example 7

### Inhibition of MDM Binding to Polyurethane Surfaces Immobilized with hCD47 and bCD47

Studies were carried out to compare the efficacy of immobilized CD47, of human (hCD47) or bovine (bCD47) origin, upon MDM binding to polyurethane (PU) surfaces. As described in Example 3, PU films were similarly modified with hCD47 or bCD47. PMA activated THP-1 cells were cultured on modified PU film surfaces. Where indicated the modified films were preincubated with anti-human CD47 antibody B6H12. Shown in Figure 7, THP-1 cell attachment was significantly reduced, compared to unmodified PU, by the presence of surface immobilized CD47 irrespective of species. Immobilized hCD47 was eight times more effective in reducing THP-1 attachment compared to bCD47. This was reversed by preincubating the hCD47 immobilized films with the anti-hCD47 antibody, which increased THP-1 binding to levels comparable with bCD47 immobilized films. The antibody showed no significant effect upon THP-1 binding to bCD47-immobilized films.

### Example 8

### Inhibition of Oxidative Degradation of Polyurethane Subdermal Implants Immobilized with hCD47 and bCD47

Rat subdermal implant model has been utilized as an *in vivo* model to investigate chronic inflammatory effects upon implanted biomaterials (Stachelek, SJ et al. (2007) J. Biomed. Mater. Res. A 82(4):1004-11). Similar to the PE degradation study described in Example 6, cracking studies of polyurethane (PU) subdermal implants were carried out in rats.

All procedures and animal husbandry were in compliance with NIH standards pertaining to the care and use of laboratory animals as approved by the IACUC of the Children's Hospital of Philadelphia. Each of the five rats per group received three 1 cm² PU-films that were composed of unmodified PU or PU surface modified with covalently appended human CD47 (hCD47) or bovine CD47 (bCD47) or a combination of the three films. Briefly, 300-350 g male Sprague-Dawley rats were anesthetized with isoflurane and administered Flunixamine post surgery for analgesia. PU implants were placed into individually dissected dorsal subdermal pouches. Animals were fed normal Purina Rat Chow ad libitum for the duration of the study. At the 70-day termination of the study, rats were euthanized by carbon dioxide asphyxiation and the subdermal implants were removed, rinsed briefly with saline and further processed as detailed below.

The rats tolerated the 10 week polyurethane (PU) subdermal implants and showed no evidence of morbidity or infection. Upon explantation all samples were readily identified and processed for assessment of evidence of oxidative related degradation. Shown in Figure 8A, Western blot analysis of the extracted proteins from hCD47 modified PU films showed the presence of hCD47 following the 10-week implantation. No immunoreactive band was observed in the extracted proteins from the unmodified PU. These results show that the CD47 modification was retained even after 10 weeks *in vivo*.

Explanted films were decellularized and assessed, via FTIR, for evidence of oxidative degradation. It has been well documented that oxidation of polyurethane elastomers corresponds with an increase in peak intensity at 1174 cm⁻¹ spectral peak (Stachelek, SJ et al. (2006) J. Biomed. Mater. Res. A. 78:653-61). The 1174 cm⁻¹ is assigned to the υ(C-O-C) of branched ether, which has been identified as a marker of oxidative degradation, and the extent of ether cross-linking can be quantitatively determined by normalizing the 1174 cm⁻¹ spectral peak intensity with the peak intensity of the 1595 cm⁻¹ spectral peak. The 1595 cm⁻¹ peak corresponds to a non-oxidized aromatic ring. Shown in Figure 8B, the immobilization of either hCD47 or bCD47 significantly reduced the aberrant cross-linking of the polyurethane's ether.

Scanning electron micrographs of the explanted films (Figures 8C-8E) showed that the most extensive surface cracking was observed on the unmodified PU surface, and the CD47 modified PU surfaces showed only slight evidence of cracks. A comparison of the cracking between the hCD47 (Figure 8D) and bCD47 (Figure 8E) modified surfaces did not show any appreciable differences.

### Example 9

### Inhibition of whole blood cellular attachment to CD47 modified polyvinyl chloride (PVC) tubing under flow conditions

The Chandler loop was used to compare the effects of the CD47 modified polyvinyl chloride (PVC) surfaces with PVC tubing either unmodified or modified with poly (2-methoxyethyl acrylate) (PMEA) (X Coating™ from Terumo) on whole blood cellular attachment under flow conditions using the protocol described in Example 5.

Figure 9 shows robust cell attachment on the surface of the unmodified PVC tubing as well as on PVC tubing that has been modified with PMEA. Morphology indicates these attached cells are not red cells, but white blood cells. There was scant evidence of cellular attachment in the CD47 modified PVC tubing. Quantitative analysis shows a 20-fold reduction in cellular attachment to the CD47 modified surfaces compared to the unmodified PVC tubing or the PMEA modified Terumo tubing (Terumo-X). These data show the anti-inflammatory effects of surface bound CD47 on PVC tubing.

Various terms relating to the systems, methods, and other aspects of the present invention are used throughout the specification and claims. Such terms are to be given their ordinary meaning in the art unless otherwise indicated.

The present invention is not limited to the embodiments described and exemplified above, but is capable of variation and modification within the scope and range of equivalents of the appended claims.

## Claims

1. A method for inhibiting or reducing immune cell attachment to a biomaterial, comprising attaching CD47 or a subdomain thereof that is capable of binding to SIRP-α, to the surface of the biomaterial, wherein the CD47 or the subdomain thereof that is capable of binding to SIRP-α inhibits or reduces immune cell-mediated damage to the biomaterial.

2. The method of claim 1, wherein the biomaterial comprises a polymer.

3. The method of claim 2, wherein the polymer is a polypropylene, polyethylene, polystyrene, polymethylmethacrylate, polyurethane, polyfluorotetraethylene, or polyvinyl, or mixtures thereof, in particular, wherein the polymer is polyurethane or polyvinyl chloride.

4. The method of claim 1, wherein the biomaterial is on the surface of an implant, on the surface of a medical device, on the surface of a tube, and/or on the surface of a therapeutic agent delivery vehicle.

5. The method of claim 4, further defined by one or more of the following:
a) the implant comprises the biomaterial, and/or the implant is coated with the biomaterial and/or wherein the implant is a pacemaker lead;
b) the medical device comprises the biomaterial and/or the medical device is coated with the biomaterial;
c) the tube comprises the biomaterial and/or the tube is coated with the biomaterial; and/or wherein the tube is part of a heart-lung bypass;
d) the therapeutic agent delivery vehicle comprises the biomaterial; and/or the therapeutic agent delivery vehicle is coated with the biomaterial.

6. The method of claim 1, wherein the CD47 or the subdomain thereof that is capable of binding to SIRP-α inhibits or reduces immune cell-mediated damage to the biomaterial *in vitro* and/or *in vivo.*

7. The method of claim 1, wherein the surface of the biomaterial comprises at least one linking molecule, wherein the linking molecule preferably is avidin, comprises a thiol, is folate or is the folate receptor.

8. The method of claim 1, wherein the CD47 or the subdomain thereof that is capable of binding to SIRP-α is complexed with a linking molecule, preferably, wherein the linking molecule is SPDP or SMCC, folate, the folate receptor or biotin.

9. The method of claim 1, wherein the immune cell expresses SIRP-α and/or, wherein the immune cell is a monocyte or macrophage.

10. The method of claim 1, wherein the damage is oxidative damage or inflammatory damage.

11. A kit for protecting a biomaterial, comprising CD47 or the subdomain thereof that is capable of binding to SIRP-α and instructions for using the kit according to the method of claim 1.

12. The kit of claim 11, further comprising
a) a linking molecule capable of being attached to the surface of the biomaterial, wherein the linking molecule is preferably avidin, in particular SPDP or SMCC or folate or the folate receptor,
b) and/or a linking molecule capable of being attached to the CD47 or the subdomain thereof that is capable of binding to SIRP-α; wherein the linking molecule is preferably biotin.

13. The kit of claim 11, wherein the CD47 or the subdomain thereof that is capable of binding to SIRP-α is complexed with
a) SPDP or SMCC
b) biotin or
c) folate.

14. The method of claim 1, wherein the immune cell is a polymorphonuclear leukocyte, in particular wherein the polymorphonuclear leukocyte is a neutrophil.

15. A biomaterial comprising CD47 or the subdomain thereof that is capable of binding to SIRP-α attached to the surface of the biomaterial.

16. The biomaterial of claim 16, further comprising at least one linking molecule on the surface of the biomaterial, wherein preferably the at least one linking molecule mediates the attachment of the CD47 or the subdomain thereof that is capable of binding to SIRP-α to the surface.

## Patentansprüche

1. Verfahren zur Hemmung oder Reduzierung von Immunzellanhaftung an Biomaterial, umfassend das Anheften von CD47 oder einer Subdomäne davon, die in der Lage ist, an SIRP-α zu binden, an die Oberfläche des Biomaterials, wobei das CD47 oder die Subdomäne davon, die in der Lage ist, an SIRP-α zu binden, Immunzell-vermittelte Schädigung des Biomaterials hemmt oder reduziert

2. Verfahren nach Anspruch 1, wobei das Biomaterial ein Polymer umfasst.

3. Verfahren nach Anspruch 2, wobei das Polymer ein Polypropylen, Polyethylen, Polystyrol, Polymethylmethacrylat, Polyurethan, Polyfluortetraethylen oder Polyvinyl oder Gemische davon ist, insbesondere wobei das Polymer Polyurethan oder Polyvinylchlorid ist.

4. Verfahren nach Anspruch 1, wobei das Biomaterial auf der Oberfläche eines Implantats, auf der Oberfläche eines Medizinprodukts, auf der Oberfläche eines Schlauchs und/oder auf der Oberfläche eines Therapeutikum-Abgabevehikels vorhanden ist.

5. Verfahren nach Anspruch 4, das weiterhin durch eines oder durch mehrere aus dem Folgenden definiert ist:
a) das Implantat umfasst das Biomaterial und/oder das Implantat ist mit dem Biomaterial beschichtet und/oder wobei das Biomaterial eine Schrittmacherelektrode ist;
b) das Medizinprodukt umfasst das Biomaterial und/oder das Medizinprodukt ist mit dem Biomaterial beschichtet;
c) der Schlauch umfasst das Biomaterial und/oder der Schlauch ist mit dem Biomaterial beschichtet und/oder wobei der Schlauch Teil eines Herz-Lunge-Bypasses ist;
d) das Therapeutikum-Abgabevehikel umfasst das Biomaterial und/oder das Therapeutikum-Abgabevehikel ist mit dem Biomaterial beschichtet.

6. Verfahren nach Anspruch 1, wobei das CD47 oder die Subdomäne davon, die in der Lage ist, an SIRP-α zu binden, Immunzell-vermittelte Schädigung des Biomaterials *in vitro* und/oder *in vivo* hemmt oder reduziert.

7. Verfahren nach Anspruch 1, wobei die Oberfläche des Biomaterials mindestens ein Verknüpfungsmolekül umfasst, wobei das Verknüpfungsmolekül vorzugsweise Avidin ist, ein Thiol umfasst, Folat oder der Folatrezeptor ist.

8. Verfahren nach Anspruch 1, wobei das CD47 oder die Subdomäne davon, die in der Lage ist, an SIRP-α zu binden, mit einem Verknüpfungsmolekül komplexiert ist, vorzugsweise wobei das Verknüpfungsmolekül SPDP oder SMCC, Folat, der Folatrezeptor oder Biotin ist.

9. Verfahren nach Anspruch 1, wobei die Immunzelle SIRP-α exprimiert und/oder wobei die Immunzelle ein Monozyt oder Makrophage ist.

10. Verfahren nach Anspruch 1, wobei die Schädigung eine oxidative Schädigung oder inflammatorische Schädigung ist.

11. Kit zum Schutz eines Biomaterials, umfassend CD47 oder die Subdomäne davon, die in der Lage ist, an SIRP-α zu binden, und Anleitungen zur Verwendung des Kits gemäß dem Verfahren nach Anspruch 1.

12. Kit nach Anspruch 11, weiterhin Folgendes umfassend:
a) ein Verknüpfungsmolekül, das in der Lage ist, an die Oberfläche des Biomaterials angeheftet zu werden, wobei das Verknüpfungsmolekül vorzugsweise Avidin, insbesondere SPDP oder SMCC oder Folat oder der Folatrezeptor ist,
b) und/oder ein Verknüpfungsmolekül, das in der Lage ist, an das CD47 oder die Subdomäne davon, die in der Lage ist, an SIRP-α zu binden, angeheftet zu werden, wobei das Verknüpfungsmolekül vorzugsweise Biotin ist.

13. Kit nach Anspruch 11, wobei das CD47 oder die Subdomäne davon, die in der Lage ist, an SIRP-α zu binden, komplexiert ist mit
a) SPDP oder SMCC
b) Biotin oder
c) Folat.

14. Verfahren nach Anspruch 1, wobei die Immunzelle ein polymorphonukleärer Leuykzyt ist, insbesondere wobei der polymorphonukleäre Leuykzyt ein Neutrophil ist.

15. Biomaterial umfassend CD47 oder die Subdomäne davon, die in der Lage ist, an SIRP-α zu binden, das an die Oberfläche des Biomaterials angeheftet ist.

16. Biomaterial nach Anspruch 16, weiterhin umfassend mindestens ein Verknüpfungsmolekül auf der Oberfläche des Biomaterials, wobei vorzugsweise das mindestens eine Verknüpfungsmolekül die Anknüpfung von CD47 oder der Subdomäne davon, die in der Lage ist, an SIRP-α zu binden, an die Oberfläche vermittelt.

## Revendications

1. Procédé permettant d'inhiber ou de réduire la fixation de cellules immunitaires à un biomatériau, comprenant la fixation d'une CD47 ou un sous-domaine de celle-ci qui est capable de se lier à SIRP-α, à la surface du biomatériau, dans lequel la CD47 ou le sous-domaine de celle-ci qui est capable de se lier à SIRP-α inhibe ou réduit les dommages induits par les cellules immunitaires au biomatériau.

2. Procédé selon la revendication 1, dans lequel le biomatériau comprend un polymère.

3. Procédé selon la revendication 2, dans lequel le polymère est un polypropylène, un polyéthylène, un polystyrène, un polyméthacrylate de méthyle, un polyuréthane, un polytétrafluoroéthylène, ou un polyvinyle, ou des mélanges de ceux-ci, en particulier, dans lequel le polymère est un polyuréthane ou un polychlorure de vinyle.

4. Procédé selon la revendication 1, dans lequel le biomatériau se trouve sur la surface d'un implant, sur la surface d'un dispositif médical, sur la surface d'un tube, et/ou sur la surface d'un véhicule d'administration d'agent thérapeutique.

5. Procédé selon la revendication 4, définie en outre par un ou plusieurs de ce qui suit :
a) l'implant comprend le biomatériau, et/ou l'implant est enduit avec le biomatériau et/ou dans lequel l'implant est un conducteur de stimulateur cardiaque ;
b) le dispositif médical comprend le biomatériau et/ou le dispositif médical est enduit avec le biomatériau ;
c) le tube comprend le biomatériau et/ou le tube est enduit avec le biomatériau ; et/ou dans lequel le tube fait partie d'un pontage cardiopulmonaire ;
d) le véhicule d'administration d'agent thérapeutique comprend le biomatériau ; et/ou le véhicule d'administration d'agent thérapeutique est enduit avec le biomatériau.

6. Procédé selon la revendication 1, dans lequel la CD47 ou le sous-domaine de celle-ci qui est capable de se lier à SIRP-α inhibe ou réduit les dommages induits par les cellules immunitaires au biomatériau *in vitro* et/ou *in vivo.*

7. Procédé selon la revendication 1, dans lequel la surface du biomatériau comprend au moins une molécule de liaison, dans lequel la molécule de liaison est de préférence une avidine, comprend un thiol, est un acide folique ou est le récepteur de l'acide folique.

8. Procédé selon la revendication 1, dans lequel la CD47 ou le sous-domaine de celle-ci qui est capable de se lier à SIRP-α est complexé avec une molécule de liaison, de préférence, dans lequel la molécule de liaison est un SPDP ou un SMCC, un acide folique, le récepteur de l'acide folique ou une biotine.

9. Procédé selon la revendication 1, dans lequel la cellule immunitaire exprime SIRP-α et/ou dans lequel la cellule immunitaire est un monocyte ou un macrophage.

10. Procédé selon la revendication 1, dans lequel les dommages sont des dommages oxydatifs ou des dommages inflammatoires.

11. Kit permettant de protéger un biomatériau, comprenant la CD47 ou le sous-domaine de celle-ci qui est capable de se lier à SIRP-α et instructions permettant d'utiliser le kit conformément au procédé selon la revendication 1.

12. Kit selon la revendication 11, comprenant en outre
a) une molécule de liaison qui est capable d'être fixée à la surface du biomatériau, dans lequel la molécule de liaison est de préférence une avidine, en particulier un SPDP ou un SMCC ou un acide folique ou le récepteur de l'acide folique,
b) et/ou une molécule de liaison qui est capable d'être fixée à la CD47 ou au sous-domaine de celle-ci qui est en mesure de se lier à SIRP-α ; dans lequel la molécule de liaison est de préférence une biotine.

13. Kit selon la revendication 11, dans lequel la CD47 ou le sous-domaine de celle-ci qui est capable de se lier à SIRP-α est complexé avec
a) un SPDP ou un SMCC
b) une biotine ou
c) un acide folique.

14. Procédé selon la revendication 1, dans lequel la cellule immunitaire est un leucocyte polynucléaire, en particulier dans lequel le leucocyte polynucléaire est un neutrophile.

15. Biomatériau comprenant la CD47 ou le sous-domaine de celle-ci qui est capable de se lier à SIRP-α fixé à la surface du biomatériau.

16. Biomatériau selon la revendication 16, comprenant en outre au moins une molécule de liaison sur la surface du biomatériau, de préférence, dans lequel la au moins une molécule de liaison induit la fixation de la CD47 ou du sous-domaine de celle-ci qui est capable de se lier à SIRP-α à la surface.
